# DEMANDE DE BREVET EUROPEEN

(11) **EP 3 566 673 A1**
(43) Date de publication de la demande: **13.11.2019**
(21) Numéro de dépôt: 18305637.3
(22) Date de dépôt: 25.05.2018
(51) Int. Cl.: A61C 7/00, A61C 9/00, A61B 1/32, G06Q 10/10, G06T 7/00, G16H 30/40, G16H 40/40, G16H 50/50, G16H 50/70, G16H 50/20, G16H 15/00, G06T 7/60

(54) **PROCEDE D'EVALUATION D'UNE SITUATION DENTAIRE**

(30) Priorité: 09.05.2018 EP 18305571
(71) Demandeur: Dental Monitoring, 75008 Paris (FR)
(72) Inventeur: SALAH, Philippe, BAGNOLET 93170 (FR); PELLISSARD, Thomas, 92110 CLICHY (FR); GHYSELINCK, Guillaume, 59169 CANTIN (FR); DEBRAUX, Laurent, 75020 PARIS (FR)
(74) Mandataire: Nony

(57) **Abrégé**

Procédé d'évaluation d'une situation dentaire d'un patient cible suivi par un professionnel des soins dentaires, ledit procédé comportant les étapes suivantes :
a) acquisition, au moyen d'un téléphone mobile, d'au moins une image actualisée représentant une arcade dentaire du patient cible ;
b) préparation, par le téléphone mobile, d'une requête comportant l'image actualisée, et transmission de la requête à un ordinateur d'analyse ;
c) analyse de la requête par l'ordinateur d'analyse, de manière à déterminer une valeur pour au moins un attribut dentaire relatif à la situation dentaire du patient cible, et transmission de la valeur de l'attribut dentaire à un ordinateur de composition ;
d) sélection, par l'ordinateur de composition, dans une base de réponses-types et en fonction de ladite valeur dudit au moins un attribut dentaire, d'au moins une réponse-type pertinente ;
e) composition, par l'ordinateur de composition, d'une réponse composée à partir de ladite au moins une réponse type pertinente ;
f) optionnellement, validation et/ou modification de la réponse composée par un contrôleur ;
g) transmission au téléphone mobile, par l'ordinateur de composition, de la réponse composée, optionnellement validée et/ou modifiée, et présentation, sur le téléphone mobile, de la réponse composée, optionnellement validée et/ou modifiée ;
la sélection à l'étape d) et la composition à l'étape e) étant déterminées conformément à des règles de composition d'un protocole de composition.

## Description

### Domaine technique

La présente invention concerne un procédé d'évaluation d'une situation dentaire.

### Etat de la technique

Il est souhaitable que chacun fasse régulièrement contrôler sa dentition, notamment afin d'évaluer sa situation dentaire, c'est-à-dire notamment de vérifier que la position ou l'aspect de ses dents n'évolue pas défavorablement ou qu'un appareil orthodontique est en bon état, ou bien positionné. Lors d'un traitement orthodontique, cette évolution défavorable peut notamment conduire à modifier le traitement. Après un traitement orthodontique, cette évolution défavorable, appelée « récidive », peut conduire à une reprise d'un traitement. Enfin, de manière plus générale et indépendamment de tout traitement, chacun peut souhaiter évaluer si sa situation dentaire, et en particulier sa situation orthodontique, est satisfaisante.

Classiquement, les contrôles sont effectués par un professionnel des soins dentaires, notamment un orthodontiste ou un dentiste, qui seul dispose d'un appareillage adapté. Ces contrôles sont donc coûteux. En outre, les visites chez le professionnel des soins dentaires sont contraignantes.

Pour limiter le nombre de ces visites, le Demandeur a développé un procédé permettant, à partir
- d'un modèle tridimensionnel d'une arcade dentaire d'un patient réalisé avant le traitement, dit « modèle de référence initial », puis
- d'une simple image «actualisée» de l'arcade prise pendant le traitement, par exemple d'une photographie prise par le patient avec son téléphone mobile, d'évaluer avec précision le positionnement des dents au moment de l'acquisition de l'image actualisée.
Ce procédé consiste en un processus itératif selon lequel, à chaque itération, des modèles de dents du modèle de référence initial sont déplacés, puis des conditions optimales d'observation du modèle initial ainsi modifié (dit « modèle de référence à tester ») sont déterminées, les conditions optimales d'observation étant définies comme les conditions permettant d'observer le modèle de référence à tester de manière que la vue dudit modèle soit la plus proche possible de l'image actualisée. On teste une succession de « modèles de référence à tester » jusqu'à obtenir un niveau de correspondance maximal entre un modèle de référence à tester et l'image actualisée. Ce dernier modèle de référence à tester est alors considéré comme représentant les dents dans leur position au moment de l'acquisition de l'image actualisée. Idéalement, ce modèle, dit « modèle de référence actualisé », est un modèle de référence tridimensionnel numérique à partir duquel l'image actualisée aurait pu être prise si ce modèle avait été réel. En pratique, il représente effectivement, avec une grande précision, les dents dans leur position au moment de l'acquisition de l'image actualisée.

La comparaison des modèles de référence initial et actualisé permet ensuite d'évaluer la situation dentaire du patient. L'évaluation de la situation dentaire est classiquement réalisée par un ordinateur centralisé, traitant à distance les requêtes envoyées par les patients avec leurs téléphones mobiles et contenant les images actualisées. Ce procédé est notamment décrit dans PCT/EP2015/074896.

Le Demandeur a également développé un procédé mettant en oeuvre un algorithme d'intelligence artificielle, de préférence un réseau de neurones, et permettant, à partir d'une simple image actualisée de l'arcade du patient, par exemple d'une photographie prise par le patient avec son téléphone mobile, d'évaluer la situation dentaire du patient. A cet effet, le réseau de neurones est préalablement entrainé au moyen d'une base d'apprentissage comportant typiquement plus de 10 000, de préférence plus de 50 000, de préférence plus de 100 000 enregistrements, chaque enregistrement contenant une image historique, de préférence une photo, d'une arcade dentaire « historique » et un descriptif « historique » fournissant des valeurs pour des attributs de l'image historique, et en particulier pour des attributs permettant d'évaluer la situation dentaire de patient. Comme le procédé décrit dans PCT/EP2015/074896, l'évaluation de la situation dentaire par intelligence artificielle est de préférence réalisée par un ordinateur centralisé, traitant à distance les requêtes envoyées par les patients avec leurs téléphones mobiles et contenant les images actualisées.

Avantageusement, les deux procédés du Demandeur décrits ci-dessus limitent le nombre de visites chez le professionnel des soins dentaires et ne nécessitent pas d'appareillage spécifique coûteux.

Cependant, les patients à qui ces procédés sont proposés ne l'utilisent pas suffisamment. En particulier, on constate que de nombreux patients cessent d'envoyer des requêtes après quelques semaines.

Il existe donc un besoin pour un procédé améliorant le taux de participation des patients.

Par ailleurs, il existe un besoin permanent pour former les patients aux bonnes pratiques d'hygiène, et en particulier au brossage des dents.

Enfin, l'évaluation de la situation dentaire réalisée par l'ordinateur centralisé n'est pas toujours bien adaptée aux besoins du professionnel de soins dentaires. En particulier, elle peut être inutile lorsque la situation dentaire est satisfaisante. Elle peut être aussi insuffisamment détaillée pour qu'il puisse établir un diagnostic ou une recommandation.

Un objectif de la présente invention est de répondre, au moins partiellement, à ces besoins.

### Résumé de l'invention

L'invention fournit un procédé d'évaluation d'une situation dentaire d'un patient cible suivi par un professionnel des soins dentaires, ledit procédé comportant les étapes suivantes :
a) acquisition, au moyen d'un téléphone mobile, d'au moins une image représentant une arcade dentaire du patient cible, dite « image actualisée » ;
b) préparation, par le téléphone mobile, d'une requête comportant l'image actualisée, et transmission de la requête à un ordinateur d'analyse ;
c) analyse de la requête par l'ordinateur d'analyse, de manière à déterminer une valeur pour au moins un attribut dentaire relatif à la situation dentaire du patient cible, et transmission de la valeur de l'attribut dentaire à un ordinateur de composition ;
d) sélection, par l'ordinateur de composition, dans une base de réponses-types et en fonction de ladite valeur dudit au moins un attribut dentaire, d'au moins une réponse-type pertinente ;
e) composition, par l'ordinateur de composition, d'une réponse composée à partir de ladite au moins une réponse type pertinente ;
f) optionnellement, validation et/ou modification de la réponse composée par un contrôleur ;
g) présentation de la réponse composée, optionnellement validée et/ou modifiée ;
la sélection à l'étape d) et la composition à l'étape e) étant déterminées conformément à des règles de composition d'un protocole de composition.

**Suivant un premier aspect principal de l'invention,** à l'étape g), l'ordinateur de composition transmet la réponse composée, optionnellement validée et/ou modifiée, au téléphone mobile, et sur le téléphone mobile présente la réponse composée, optionnellement validée et/ou modifiée.

Comme on le verra plus en détail dans la suite de la description, un procédé selon le premier aspect principal de l'invention permet de répondre de manière personnalisée, mais automatique, au patient cible.

En particulier, les inventeurs ont découvert que des patients cessent d'envoyer des requêtes quand ils ne reçoivent pas de réponse et que les professionnels des soins dentaires ne répondent pas toujours à toutes les requêtes qu'ils reçoivent. En systématisant la procédure de réponse, le taux de participation des patients augmente sensiblement.

Les inventeurs ont également observé que l'insertion de conseils dans les réponses composées permet avantageusement d'éduquer les patients aux bonnes pratiques, et en particulier d'améliorer l'hygiène bucco-dentaire.

Le protocole de composition peut être commun à tous les patients d'un groupe de patients du professionnel des soins dentaires ou être spécifique au patient.

**Suivant un deuxième aspect principal de l'invention,** à l'étape g), l'ordinateur de composition transmet et présente au professionnel de soins dentaires la réponse composée, optionnellement validée et/ou modifiée.

La réponse composée peut avantageusement consister en un bilan médical, et de préférence comporter des conseils pour le professionnel de soins dentaires, adaptés au bilan médical.

Comme on le verra plus en détail dans la suite de la description, un procédé selon le deuxième aspect principal de l'invention permet de répondre de manière personnalisée, mais automatique, au professionnel de soins dentaires. En définissant le protocole de composition, le professionnel de soins dentaires peut par exemple :
- décider des seuils à partir desquels il souhaite qu'une réponse composée lui soit transmise, et/ou
- définir le niveau de détail qu'il souhaite dans la réponse composée, et/ou
- décider si la réponse composée doit comporter des préconisations médicales, et/ou
- décider des informations qu'il souhaite mettre en évidence dans la réponse composée, et/ou
- définir un ou plusieurs autres destinataires de la réponse composée, par exemple pour accélérer la commande d'un article orthodontique ou préparer le prochain rendez-vous avec le patient cible.

Un procédé selon l'invention peut encore comporter une ou plusieurs des caractéristiques optionnelles suivantes :
- la valeur de l'attribut dentaire qualifie ou quantifie la situation dentaire générale du patient cible et/ou une situation orthodontique ;
- la valeur de l'attribut dentaire qualifie ou quantifie un état d'un appareil orthodontique représenté sur l'image actualisée et/ou un état d'une dent du patient cible représentée sur l'image actualisée et/ou l'hygiène bucco-dentaire du patient ;
- avant l'étape e), le professionnel des soins dentaires sélectionne le protocole de composition dans une base de protocoles paramétrés et/ou crée le protocole de composition en sélectionnant un protocole de composition dans une base de protocoles paramétrés puis en le modifiant ;
- avant l'étape e), le professionnel des soins dentaires configure le protocole de composition de manière à sélectionner une région de la bouche du patient pour que l'analyse à l'étape c) et/ou la sélection de la réponse-type pertinente à l'étape d) ne portent que sur ladite région ;
- à l'étape d), l'ordinateur sélectionne au moins une réponse-type en fonction de données relatives au patient cible non déterminables par l'analyse de la situation dentaire de l'étape c) ;
- avant l'étape d), l'ordinateur de composition évalue
   - la réaction du patient cible, en particulier suivant le premier aspect principal de l'invention, et/ou
   - la réaction du professionnel de soins dentaires, en particulier suivant le deuxième aspect principal de l'invention, suite à l'envoi d'une réponse composée antérieure générée lors d'une étape g) antérieure, puis, à l'étape d), sélectionne ladite réponse-type en fonction de l'évaluation de ladite réaction ;
- la réponse composée antérieure est une réponse générée lors d'un cycle d'étapes a) à g) antérieur, à partir d'une image actualisée antérieure, ou est une réponse générée lors d'une étape g) antérieure portant sur ladite image actualisée ;
- au moins une règle de composition est configurée pour agir sur ladite composition en fonction de la dite au moins une réponse type pertinente sélectionnée à l'étape d), et/ou en fonction de données relatives au patient cible et/ou en fonction de données relatives au professionnel de soins dentaires ;
- la règle de composition est configurée pour agir sur ladite composition en fonction de données relatives au patient cible non déterminables par l'analyse de la situation dentaire de l'étape c) ;
- à l'étape f), le contrôleur est un professionnel des soins dentaires identique ou différent du professionnel des soins dentaires qui suit le patient cible ;
- dans un mode de réalisation, l'ordinateur d'analyse et/ou l'ordinateur de composition sont intégrés dans le téléphone mobile ;
- dans un mode de réalisation, l'ordinateur d'analyse et/ou l'ordinateur de composition sont à distance du téléphone mobile, et, en particulier peuvent être dans un centre d'évaluation avec lequel le téléphone mobile communique, le centre d'évaluation traitant informatiquement, de préférence, des requêtes en provenance de patients de plus de 1, plus de 5, plus de 50, plus de 100, de préférence plus de 1000 professionnels de soins dentaires ;
- suivant le premier aspect principal de l'invention, l'ordinateur de composition compose de préférence, suivant un protocole de composition identique ou différent de celui mis en oeuvre à l'étape d) et à l'étape e), un bilan médical en fonction de ladite valeur dudit au moins un attribut dentaire, et présente ledit bilan médical au professionnel de soins dentaires.

Bien entendu, les deux aspects principaux de l'invention ne sont pas incompatibles. En particulier, le procédé peut être utilisé, pour une même situation dentaire, pour générer une réponse composée destinée au patient cible et une autre réponse composée destinée au professionnel de soins dentaires, de préférence suivant deux protocoles de composition différents. Par souci de clarté, dans la présente description, on qualifie de « professionnel » le protocole de composition utilisé pour générer une réponse composée destinée au professionnel de soins dentaires.

### Définitions

La « représentation d'une arcade dentaire» est une représentation de tout ou partie de ladite arcade.

Par « patient cible », on entend toute personne pour laquelle un procédé selon l'invention est mis en oeuvre afin d'en évaluer la situation dentaire, que cette personne soit malade ou non.

Par « téléphone mobile », on entend tout appareil portable permettant de prendre des photos ou des films et de communiquer par un réseau de téléphonie mobile.

Par « ordinateur », on désigne une unité de traitement informatique. Cette unité peut être notamment intégrée dans le téléphone mobile ou être un ordinateur de type PC ou un serveur d'un « centre d'évaluation », par exemple un ordinateur disposé chez le professionnel des soins dentaires. Le téléphone mobile et l'ordinateur comportent alors des moyens de communication pour échanger entre eux, notamment pour transmettre la requête comportant l'image actualisée et/ou la réponse composée.

Par "image", on entend une image en deux dimensions, comme une photographie ou une image d'une vidéo. Une image est formée de pixels.

"Comprendre", "comporter" et "présenter" doivent être interprétés de manière large et non limitative, sauf indication contraire.

### Brève description des figures

D'autres caractéristiques et avantages de l'invention apparaîtront encore à la lecture de la description détaillée qui va suivre et à l'examen du dessin annexé dans lequel :

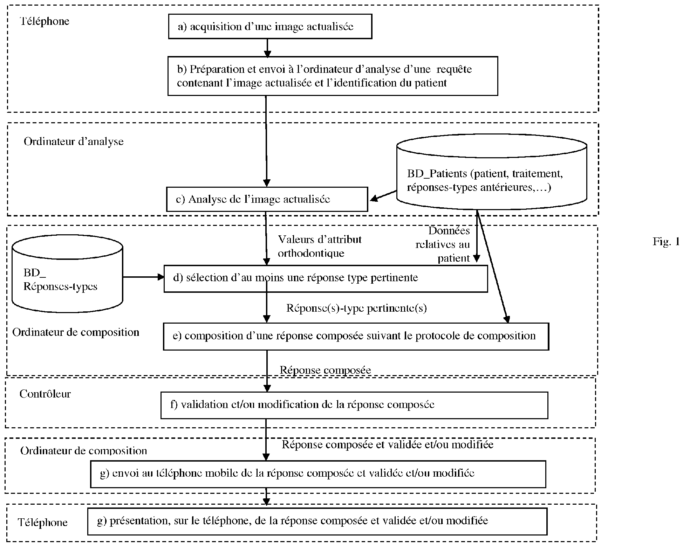

- la figure 1 illustre la mise en oeuvre d'un procédé selon l'invention dans un mode de réalisation dans lequel l'analyse de la requête et la composition de la réponse composée sont effectuées à distance du téléphone mobile, et
- la figure 2 illustre l'utilisation d'un écarteur ;

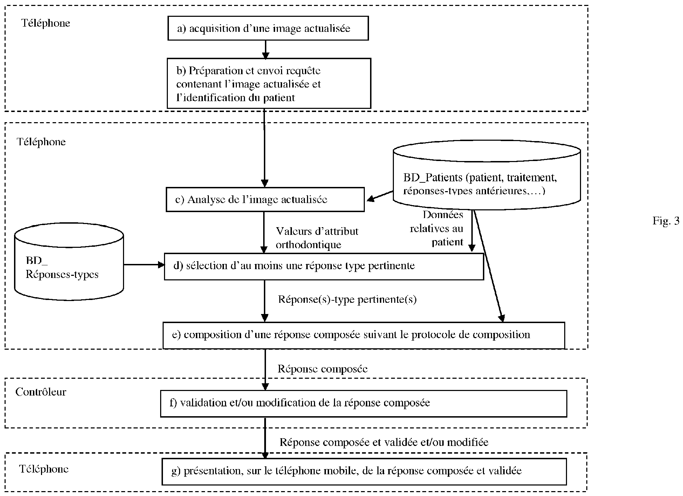

- la figure 3 illustre la mise en oeuvre d'un procédé selon l'invention dans un mode de réalisation dans lequel l'analyse de la requête et la composition de la réponse composée sont effectuées dans le téléphone mobile.

### Description détaillée d'un premier mode de réalisation

Un procédé selon l'invention comporte les étapes mentionnées ci-dessus. Ce procédé est décrit en détail dans le cadre du premier aspect principal de l'invention, pour présenter une réponse composée au patient cible.

Le procédé a alors pour objectif de générer automatiquement des réponses aux requêtes des patients. A cet effet, le procédé utilise un protocole de composition, applicable au patient cible ou à un groupe de patients du professionnel des soins dentaires, et qui fixe les règles de composition régissant l'étape e).

De préférence, le groupe de patients, ou « cohorte », comporte plus de 20, plus de 50, plus de cent, plus de trois cents, plus de cinq cents, voire plus de mille patients. Dans un mode de réalisation, le groupe de patients comporte tous les patients suivis par le professionnel des soins dentaires. Avantageusement, le professionnel des soins dentaires peut ainsi appliquer la même procédure quel que soit le patient considéré.

La cohorte peut également regrouper les patients ayant les mêmes pathologies, par exemple tous les patients souffrant de l'apnée du sommeil.

Le protocole de composition comporte un ensemble de règles de composition qui déterminent la sélection des réponses-types pertinentes, ainsi que la façon dont les réponses-types pertinentes sélectionnées sont modifiées et/ou agencées pour composer la réponse composée.

La base de réponses-types dans laquelle sont sélectionnées les réponses-types pertinentes peut comporter plus de 100, plus de 500 ou plus de 1000 réponses-types.

En particulier, elle peut comporter des messages fournissant une information sur la situation dentaire du patient cible, des messages fournissant une préconisation, par exemple relative au brossage d'une dent particulière, des messages de félicitation ou d'encouragement, des messages d'avertissement, des messages rassurants ou des messages demandant au patient cible de prendre rendez-vous avec le professionnel des soins dentaires.

Pour la sélection des réponses-types pertinentes, une règle de composition peut être un simple filtre déterminant si une réponse-type associée peut être intégrée ou non dans la réponse composée.

Pour la modification des réponses-types pertinentes, une règle de composition peut par exemple imposer un format particulier à une réponse-type sélectionnée, par exemple imposer un fond de page, une police de caractères, une taille de caractère ou une couleur de caractères, ou imposer une traduction dans une langue particulière, par exemple dans une langue connue du patient-cible, ou imposer un registre de langage particulier.

Pour l'agencement des réponses-types pertinentes, éventuellement modifiées, une règle de composition peut être de simplement afficher toutes les réponses-types pertinentes, par exemple dans un courriel. Une règle de composition peut également déterminer la position de chaque réponse-type pertinente en fonction de son contenu ou de la catégorie à laquelle elle appartient.

Une règle de composition peut s'appliquer à une réponse-type associée, à un ensemble de réponses-types associé, c'est-à-dire à une « catégorie » de réponses-types, à toutes les réponses-types sélectionnées, ou à la réponse composée.

Une règle de composition peut être conditionnelle ou non conditionnelle.

En particulier, son action peut dépendre de données relatives au patient cible, et en particulier de sa situation dentaire, notamment orthodontique (ou « situation clinique »).

Son action peut aussi dépendre d'une ou plusieurs autres réponses-types sélectionnées pour répondre à la réception de l'image actualisée et/ou d'une ou plusieurs réponse-types sélectionnées pour composer une réponse composée qui a été envoyée antérieurement au patient cible et/ou de la réaction du patient-cible à une ou plusieurs de réponses composées antérieures qui lui ont été envoyées antérieurement, de préférence dans le même cadre orthodontique (même période entre deux traitements orthodontiques ou même traitement orthodontique).

L'action d'une règle de composition peut également dépendre des réactions d'un ou plusieurs autres patients « historiques » à une ou plusieurs de réponses composées antérieures, générées conformément à l'invention, qui leur ont été envoyées antérieurement, de préférence dans le cadre de traitements orthodontiques sensiblement identiques à celui du patient cible.

L'action d'une règle de composition peut encore dépendre de caractéristiques du patient cible indépendantes de sa situation dentaire, par exemple de son âge ou de son sexe, ou de la proximité affective entre le patient cible et le professionnel des soins dentaires, notamment pour déterminer si le tutoiement ou le vouvoiement doit être utilisé.

L'action d'une règle de composition peut encore dépendre de l'historique du patient cible, c'est-à-dire par exemple des traitements qu'il a subis ou des requêtes qu'il a envoyées par le passé ou de son comportement lors des traitements. Elle peut en particulier dépendre de la valeur, déterminée lors d'au moins une étape c) antérieure, pour au moins un attribut dentaire relatif à la situation dentaire du patient cible, c'est-à-dire de l'analyse d'images actualisées d'autres cycles d'étapes a) à f).

L'action d'une règle de composition peut aussi dépendre de données indépendantes du patient cible, ou « données extérieures ». Par exemple, elle peut dépendre de la période de l'année à laquelle la requête du patient cible a été reçue, par exemple pour souhaiter une bonne année au début du mois de janvier.

Elle peut aussi dépendre d'un choix arbitraire du professionnel de soins dentaires, en particulier du choix qui lui est propre de communiquer à propos d'une situation dentaire particulière ou d'une région particulière de la bouche du patient.

Les exemples suivants illustrent la variété des règles de composition possibles :
Une règle de composition qui dépend de la situation dentaire du patient cible, c'est-à-dire de la valeur d'au moins un attribut dentaire, peut être par exemple : *« si la valeur d'attribut dentaire est « gouttière orthodontique décollée de la dent n°13 », sélectionner la réponse-type pertinente suivante : 'Les images que vous nous avez transmises nous ont permis de détecter un décollement de votre gouttière orthodontique sur une canine de droite, sur l'arcade supérieure' ».*

Une règle de composition dont l'effet dépend de la réponse-type à laquelle elle s'applique peut être par exemple : « *imposer une couleur rouge pour les seules réponses-types pertinentes relatives à des avertissements ».*

Une règle de composition modifiant une réponse-type pertinente ou la réponse composée dans son ensemble peut être par exemple : *« si le patient cible est anglais, utiliser exclusivement des réponses-types en anglais »,* ou « *si le patient cible est anglais, traduire la réponse composée en anglais ».*

D'autres règles de composition conditionnelles tenant compte des évènements passés peuvent être par exemple : « *si le patient cible n'a pas répondu au rappel précédent, cette réponse-type ne peut être sélectionnée comme réponse-type pertinente* » ou *« si la réponse-type n°8 a été sélectionnée comme réponse-type pertinente pour composer la réponse composée précédente, la réponse-type n°9 ne peut être sélectionnée comme réponse-type pertinente ».*

Une règle de composition qui s'applique à la réponse composée et qui dépend de données relatives au patient peut être par exemple : « *si le patient cible est un homme, introduire la réponse composée par 'Cher Monsieur*' *suivi du nom du patient cible, en minuscules, avec une première lettre en majuscule».*

Une règle de composition dont l'effet dépend des échanges antérieurs avec le patient cible peut être par exemple *« si le patient cible n'a pas pris rendez-vous en dépit de deux relances successives, sélectionner la réponse-type pertinente 'Il est absolument indispensable que vous preniez rapidement rendez-vous. Votre situation dentaire se dégrade' ».*

Une règle de composition dont l'effet dépend d'un choix arbitraire du professionnel de soins dentaires peut être par exemple : *« ne pas intégrer, dans la réponse composée, d'alerte relative aux interférences occlusales »,* ou *« ne pas intégrer, dans la réponse composée, d'alerte relative à la dent n°12 ».*

Dans un mode de réalisation, le protocole de composition est unique et figé. De préférence cependant le professionnel des soins dentaires peut le choisir dans une base de modèles de protocole et/ou en modifier les valeurs des paramètres des règles de composition.

Dans un mode de réalisation, au moins une partie des modèles de protocole sont pré-paramétrés et le professionnel de soins dentaires modifie les valeurs des paramètres, par exemple en fonction du type de patient et/ou de choix qui lui sont spécifiques et/ou de spécificités du patient. Par exemple, il peut utiliser un modèle de protocole pré-paramétré pour un adulte et en modifier des valeurs de paramètres pour qu'il soit adapté à un enfant.

Les choix peuvent être spécifiques au professionnel de soins dentaires. Par exemple, selon les professionnels de soins dentaires, le protocole de composition devra signaler l'apparition de malocclusion ou non. Des valeurs de paramètres peuvent donc être indépendantes du patient et du traitement et relever d'une décision arbitraire du professionnel de soins dentaires.

Pour paramétrer les valeurs de paramètres, une case à cocher peut être associée à chaque réponse-type de la base de réponses-types, l'activation ou la désactivation de cette case définissant si la réponse-type peut être ou non utilisée comme réponse-type pertinente. La valeur de cette case à cocher est un exemple de valeur d'un paramètre d'une règle de composition définissant la possibilité ou l'interdiction d'utiliser la réponse-type.

De préférence encore, les réponses-types sont classées dans des catégories. De préférence, au moins un paramètre concerne toutes les réponses-types d'une catégorie, c'est-à-dire que la valeur de ce paramètre est applicable à l'ensemble des réponses-types de cette catégorie. Le paramétrage par le professionnel des soins dentaires en est avantageusement simplifié.

De préférence, lorsque le professionnel des soins dentaires peut choisir le protocole de composition dans une base de modèles de protocole, les modèles de protocole comportent des valeurs par défaut pour chacun des paramètres et le professionnel des soins dentaires peut modifier les valeurs de paramètres.

De préférence, le professionnel de soins dentaires peut paramétrer le protocole de composition pour sélectionner une région de la bouche sur laquelle l'analyse à l'étape c) et/ou la composition à l'étape e) doivent porter. En particulier, de préférence, le protocole de composition est configuré pour autoriser une sélection des dents.

Avantageusement, la réponse composée ne comportera donc pas d'information sur d'autre partie de la bouche que ladite région. En outre, l'analyse à l'étape c) en sera accélérée.

Avant l'étape a), un rappel informe de préférence le patient cible de la nécessité d'acquérir une image actualisée. Ce rappel est envoyé sur son téléphone mobile, par exemple sous la forme d'un courriel, d'une alerte ou d'un SMS.

**A l'étape a),** une image actualisée de l'arcade dentaire du patient cible est acquise au moyen d'un téléphone mobile. L'étape a) est de préférence réalisée par le patient cible ou un proche du patient cible. L'étape a) peut avantageusement être réalisée à distance du centre d'évaluation, en particulier à plus de 1 km, ou plus de 5 km du lieu du centre d'évaluation, en particulier hors cabinet orthodontique.

De préférence, l'image actualisée est une photographie, en particulier une photographie panoramique. Dans un mode de réalisation, l'image actualisée est extraite d'un film.

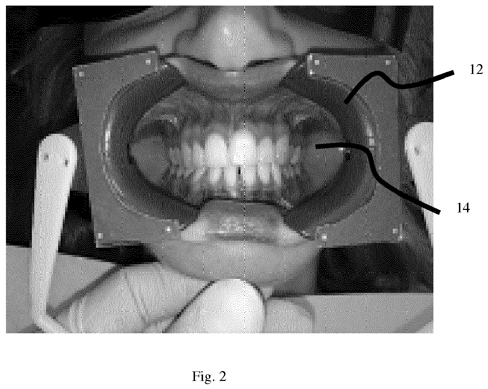

De préférence, on utilise un écarteur lors de l'étape a), comme représenté sur la figure 2. Un écarteur est un dispositif bien connu, comportant des goulottes 12 s'étendant autour d'une ouverture 14. Le patient cible dispose ses lèvres dans les goulottes, ce qui permet de les écarter et d'améliorer l'observation des arcades dentaires à travers l'ouverture. De préférence, un écarteur est remis au patient cible, par exemple lors d'un rendez-vous avec son orthodontiste.

**A l'étape b),** une requête est préparée par un applicatif chargé dans le téléphone mobile. Cette requête comporte une image actualisée acquise à l'étape a), mais également des informations permettant d'identifier l'origine de la requête, c'est-à-dire un identifiant du patient cible « ID_patient ».

La requête est traitée par un « ordinateur d'analyse », qui peut notamment être un module de traitement intégré dans le téléphone mobile ou être un ordinateur d'un centre d'évaluation à distance du téléphone mobile.

Dans ce dernier cas, la requête est transmise à l'ordinateur d'analyse par le téléphone mobile. Tous les moyens de communication connus peuvent être utilisés à cet effet. La transmission peut être en particulier effectuée par un réseau de téléphonie mobile auquel le téléphone mobile est abonné.

L'ordinateur d'analyse peut recevoir les requêtes provenant d'une pluralité de patients cibles du professionnel des soins dentaires, et, en particulier lorsqu'il est dans un centre d'évaluation, de préférence de plusieurs professionnels des soins dentaires. De préférence, il reçoit des requêtes de plus de 1000, plus de 10 000, de préférence plus de 50 000, de préférence encore plus de 100 000 patients cibles.

**A l'étape c),** l'ordinateur d'analyse analyse la requête. En particulier, il analyse l'image actualisée afin d'évaluer la situation dentaire du patient cible. Dans un mode de réalisation, l'évaluation de la situation dentaire du patient cible est validée et/ou modifiée, notamment complétée, par un professionnel des soins dentaires. Le professionnel des soins dentaires peut également agir en conséquence, par exemple pour commander une nouvelle gouttière orthodontique lorsque l'évaluation indique que celle qui est portée par le patient cible n'est plus adaptée.

L'analyse de l'image actualisée peut en particulier mettre en oeuvre des procédés d'optimisation, et notamment des méthodes métaheuristiques, comme décrit dans PCT/EP2015/074896. Le procédé décrit dans ce document lui permet notamment de déterminer les déplacements éventuels des dents représentées sur l'image actualisée, et/ou des modifications de leur forme ou de leur aspect. L'analyse peut également utiliser des algorithmes d'intelligence artificielle, et notamment un réseau de neurones utilisant une base d'apprentissage. Ces algorithmes sont bien connus.

Dans un mode de réalisation, l'analyse n'utilise que les informations fournies par l'image actualisée.

Dans un mode de réalisation préféré, l'analyse effectuée à l'étape c) utilise non seulement l'information contenu dans l'image actualisée, mais également des données relatives au patient cible, extraites d'une base de données « BD_patients » à partir de l'identifiant du patient cible déterminé à partir de la requête. L'analyse en est avantageusement plus précise.

La base des données relatives aux patients « BD_patients » peut en particulier contenir des informations générales sur chaque patient, par exemple son âge, des informations relatives au traitement en cours ou à des traitements subis par le passé, et des informations relatives à des échanges antérieurs entre le patient et l'ordinateur d'analyse.

L'analyse de l'image actualisée permet de déterminer une valeur pour au moins un attribut relatif à la situation dentaire du patient cible, ou « attribut dentaire ».

L'attribut dentaire peut être relatif à la situation dentaire générale, et par exemple prendre les valeurs « situation dentaire satisfaisante » ou « situation dentaire insatisfaisante », relatif à un état d'un appareil orthodontique représenté sur l'image actualisé, et prendre par exemple les valeurs « appareil intact » ou « appareil endommagé », relatif à l'état d'une dent, et prendre par exemple les valeurs « dent abrasée », « dent cassée », ou « dent cariée ».

Les valeurs d'un attribut dentaire peuvent être également quantitatives. Par exemple, une valeur d'un attribut peut définir la mesure dans laquelle une dent a bougé, a été abrasée ou a changé d'apparence.

Les valeurs des attributs dentaires sont alors transmises, par l'ordinateur d'analyse, à un « ordinateur de composition », qui peut être identique ou différent de l'ordinateur d'analyse. Tous les moyens de communication connus peuvent être utilisés à cet effet.

L'ordinateur de composition peut être également intégré dans le téléphone mobile.

**A l'étape d),** l'ordinateur de composition sélectionne une ou plusieurs réponses-types pertinentes au regard de la situation dentaire. A cet effet, il recherche dans une base de réponses-types, « BD_réponses-types », une ou plusieurs réponses-types répondant aux valeurs d'attribut dentaire déterminées à l'étape c) et aux règles de composition.

Les règles de composition permettent de préférence une sélection en fonction de données relatives au patient cible, et en particulier relatives au traitement du patient cible.

Par exemple, si une valeur d'attribut dentaire indique un décollement d'une gouttière orthodontique, un tel décollement peut être interprété de manière différente en fonction de l'efficacité du traitement en cours. Par exemple, si les données relatives au patient cible indiquent que le décollement est apparu très rapidement, l'ordinateur peut également sélectionner la réponse-type pertinente : « *Nous vous remercions de prendre un rendez-vous de toute urgence* ». Si au contraire, ce décollement fait suite à une évolution très lente de la position des dents, l'ordinateur peut sélectionner la réponse-type pertinente : *« Nous vous remercions de prendre un rendez-vous avec l'orthodontiste dans les deux mois ».*

Plusieurs réponses-types pertinentes peuvent être sélectionnées pour un même aspect de la situation dentaire. Par exemple, une première réponse-type pertinente peut être relative au décollement de la gouttière et une deuxième réponse-type pertinente peut être relative à une déformation anormale de cette gouttière.

Des réponses-types pertinentes différentes peuvent être sélectionnées pour différents aspects de la situation dentaire. Par exemple, une réponse-type pertinente peut être relative au décollement de la gouttière orthodontique et une deuxième réponse-type pertinente peut être relative à l'apparition d'une carie.

**A l'étape e),** une réponse est composée par l'ordinateur de composition, suivant le protocole de composition, à partir de la ou des réponses-types pertinentes déterminées à l'étape d).

L'étape de composition peut être limitée à la préparation d'un message, par exemple d'un SMS ou d'un courriel reprenant littéralement la ou les réponses-types pertinentes.

La composition de la réponse composée peut aussi comprendre des opérations de mise en forme ou de formatage, par exemple pour introduire une formule de politesse ou la signature du professionnel des soins dentaires qui suit le patient cible.

La composition de la réponse composée peut aussi comporter une opération d'agencement des différentes réponses-types pertinentes reçues, et l'introduction de phrases de liaison entre ces différentes réponses-types pertinentes.

Les données relatives au patient cible peuvent être également utilisées pour modifier la ou les réponses-types afin qu'elles soient mieux adaptées au patient cible, par exemple traduites dans une langue comprise du patient cible.

De préférence, l'étape e) fournit une réponse proche de celle qu'un professionnel des soins dentaires aurait pu rédiger, de préférence dans un registre de langue adapté au patient cible.

**A l'étape f),** la réponse composée par l'ordinateur de composition est de préférence soumise à un contrôleur afin qu'il la valide ou la modifie, par exemple en la complétant. Le contrôleur est un professionnel des soins dentaires qui peut être identique ou différent de celui qui suit le patient cible. En particulier, dans un mode de réalisation préféré, le contrôleur contrôle et/ou modifie des réponses composées pour des patients de plus de 1, plus de 5, plus de 10, voire plus de 50 professionnels des soins dentaires. Dans un mode de réalisation, le contrôleur n'a pas de patientèle propre.

Dans un mode de réalisation, la réponse-type pertinente est ou n'est pas vérifiée par un contrôleur, selon la réponse-type pertinente.

**A l'étape g),** si l'ordinateur de composition n'est pas intégré dans le téléphone mobile, la réponse composée est envoyée au téléphone mobile par l'ordinateur de composition, de préférence au moyen d'un réseau de téléphonie mobile.

La réponse composée est ensuite présentée sur le téléphone mobile. Avantageusement, le patient cible reçoit donc rapidement une réponse à sa requête, bien adaptée à sa situation dentaire.

De préférence, une réponse composée est présentée systématiquement au patient, pour chaque occurrence de l'étape a).

De préférence encore, un message d'information est également présenté au professionnel de soins dentaires qui suit le patient. Le message d'information peut être une copie de la réponse composée ou être personnalisée, suivant le deuxième aspect principal de l'invention. Un procédé selon l'invention permet ainsi, avantageusement, au professionnel d'obtenir systématiquement un compte-rendu de la situation dentaire du patient et d'agir en conséquence.

Le procédé peut aussi servir pour informer le patient afin qu'il améliore son hygiène bucco-dentaire, par exemple pour lui apprendre à mieux se brosser les dents.

Comme cela apparaît clairement à présent, la réponse composée dépend donc de :
- de l'analyse de l'image actualisée ;
- de la liste des réponses-types disponibles dans la base des réponses-types ;
- du protocole de composition.

Un procédé selon l'invention permet ainsi de répondre à une interrogation portant sur la situation dentaire d'un patient cible de manière rapide, sans contrainte pour le professionnel des soins dentaires, et précise.

Le procédé peut notamment être mis en oeuvre pour contrôler le déroulement d'un traitement orthodontique, après la pose d'un appareil destiné à corriger le positionnement des dents du patient cible, dit « appareil orthodontique actif ».

Le procédé peut être également mis en oeuvre après un traitement orthodontique, pour vérifier que le positionnement des dents n'évolue pas de façon défavorable (« récidive »), notamment après la pose d'un appareil destiné maintenir les dents en position, dit « appareil orthodontique passif ».

### Exemple

Dans un mode de réalisation préféré, un modèle de protocole pré-paramétré est présenté au professionnel de soins dentaires lorsqu'il veut appliquer un protocole de composition pour composer et adresser une réponse composée à un patient cible spécifique.

De préférence, le modèle de protocole liste des réponses-types classées, par exemple, en fonction du cadre dans lequel la patient cible est suivi, par exemple en fonction de l'appareil orthodontique éventuellement porté par le patient (gouttière orthodontique, appareil à arc et attaches, appareil de contention,...) ou hors traitement orthodontique (évaluation intra-orale générale).

De préférence, les réponses-types sont encore classées, au sein de ces catégories, dans des sous-catégories, par exemple en fonction de l'élément de l'appareil orthodontique concerné. Par exemple, dans la catégorie relative aux appareils orthodontiques à arcs et attaches, les réponses-types peuvent être classées, dans des sous-catégories « arc » et « attaches » selon qu'elles portent sur l'arc ou sur les attaches, respectivement.

De préférence, les réponses-types sont encore classées, au sein de ces sous-catégories, dans des rubriques, par exemple en fonction de la nature de l'anomalie. Par exemple, dans la sous-catégorie relative aux attaches, les réponses-types peuvent être classées, dans des rubriques « attache décollée » et « attache endommagée » selon qu'elles portent sur un décollement ou un endommagement de l'attache, respectivement.

Pour chaque réponse-type, le professionnel de soins dentaires peut de préférence :
- choisir le niveau d'alerte, par exemple choisir de ne rien faire, d'informer le patient cible, ou d'alerter le patient avec l'ajout d'un indicateur d'importance haute ; et/ou
- choisir les dents à exclure de l'analyse à l'étape c), et donc pour lesquelles la réponse composée ne comportera pas d'information ; et/ou
- choisir un message pour le patient cible, de préférence dans une liste prédéfinie ; et/ou
- choisir un message pour le professionnel de soins dentaires, de préférence dans une liste prédéfinie.

De préférence, indépendamment des réponses-types, le modèle de protocole pré-paramétré permet également au professionnel de soins dentaires de :
- définir la fréquence à laquelle des scans seront demandés au patient en l'absence d'instructions spécifiques adressées au patient cible par le professionnel de soins dentaires (étape a)) ;
- définir la fréquence à laquelle des scans seront demandés au patient en cas d'instructions spécifiques adressées au patient cible par le professionnel de soins dentaires, par exemple pour lui indiquer qu'il doit changer de gouttière orthodontique (étape a)).

### Description détaillée d'un deuxième mode de réalisation

Un procédé selon le deuxième aspect principal de l'invention peut comporter une ou plusieurs des caractéristiques optionnelles du procédé selon le premier aspect principal de l'invention décrit ci-dessus, sauf incompatibilité.

Un procédé selon le deuxième aspect principal de l'invention est destiné à présenter au professionnel de soins dentaires une réponse composée grâce à un protocole de composition « professionnel » identique ou, de préférence, différent de celui mis en oeuvre pour composer la réponse composée destinée au patient cible.

De préférence, le protocole de composition professionnel permet de définir des seuils à partir desquels une réponse composée doit être composée et transmise au professionnel de soins dentaires. Par exemple, le protocole de composition professionnel permet de déterminer si une situation dentaire, par exemple une malocclusion non pathologique, doit être signalée ou non au professionnel de soins dentaires.

De préférence, le protocole de composition professionnel permet de définir un niveau de détail pour la réponse composée, par exemple pour préciser des valeurs de déplacement ou de déformation des dents.

De préférence, le protocole de composition professionnel permet de déterminer si la réponse composée doit comporter des préconisations médicales, par exemple pour conseiller une démarche particulière comme la réalisation d'un cliché par tomographie, ou la modification d'un appareil orthodontique, ou le changement d'appareil orthodontique du patient cible, ou plus généralement la modification du traitement.

Dans un mode de réalisation, la réponse composée destinée au professionnel de soins dentaires ne fournit pas un diagnostic et/ou ne comporte aucune préconisation médicale.

De préférence, le protocole de composition professionnel permet de déterminer si la réponse composée doit comporter des préconisations pour modifier le planning des rendez-vous avec le patient cible et/ou modifier le protocole de composition utilisé pour composer les réponses composées à destination du patient cible et/ou modifier la fréquence des rappels adressés au patient cible pour qu'il mette en oeuvre une étape a).

De préférence, le protocole de composition professionnel permet aussi de déterminer le format de la réponse composée, comme décrit précédemment.

De préférence, le protocole de composition professionnel permet aussi de définir une liste de destinataires de la réponse composée, voire définir des documents spécifiques à envoyer aux destinataires.

De préférence, le protocole de composition professionnel permet aussi d'informer le professionnel de soins dentaires qu'il doit engager de nouvelles tâches. Par exemple, il peut conduire à composer une réponse composée permettant d'informer une assistante dentaire qu'elle doit préparer la séance de soins suivante en conséquence des valeurs d'attribut dentaire déterminées à l'étape c). L'assistante médicale peut par exemple être informée qu'elle doit préparer un plateau pour la séance de soins suivante, notamment pour y disposer le matériel de soin consommable nécessaire à cette séance.

De préférence, le protocole de composition professionnel permet aussi d'engager automatiquement de nouvelles tâches. Par exemple, le protocole de composition professionnel peut conduire à la génération d'un courrier ou d'un mail à un prothésiste pour qu'il fabrique une prothèse, ou au patient cible afin qu'il prenne rendez-vous. Le protocole de composition professionnel peut également, par exemple, conduire à la génération d'une commande de pièces d'appareil orthodontique, par exemple des attaches, et/ou d'un appareil orthodontique, par exemple d'une gouttière.

Le procédé peut servir à assurer un contrôle du patient cible, en particulier en dehors des périodes de traitement orthodontique ou de soins dentaires. Les problèmes dentaires, par exemple un début de carie ou de nécrose d'un tissu mou ou d'infection, peuvent donc être détectés de manière précoce, voire être anticipés, en particulier si l'analyse à l'étape c) met en oeuvre de l'intelligence artificielle.

La mise en oeuvre d'un protocole de composition professionnel permet au professionnel de soins dentaires de gagner du temps, mais encore d'obtenir un bilan médical bien adapté à ses préférences.

Bien entendu, l'invention n'est pas limitée aux modes de réalisation décrits ci-dessus et représentés.

En particulier, le patient cible n'est pas limité à un être humain. Un procédé selon l'invention peut être utilisé pour un autre animal.

## Revendications

1. Procédé d'évaluation d'une situation dentaire d'un patient cible suivi par un professionnel des soins dentaires, ledit procédé comportant les étapes suivantes :
a) acquisition, au moyen d'un téléphone mobile, d'au moins une image actualisée représentant une arcade dentaire du patient cible ;
b) préparation, par le téléphone mobile, d'une requête comportant l'image actualisée, et transmission de la requête à un ordinateur d'analyse ;
c) analyse de la requête par l'ordinateur d'analyse, de manière à déterminer une valeur pour au moins un attribut dentaire relatif à la situation dentaire du patient cible, et transmission de la valeur de l'attribut dentaire à un ordinateur de composition ;
d) sélection, par l'ordinateur de composition, dans une base de réponses-types et en fonction de ladite valeur dudit au moins un attribut dentaire, d'au moins une réponse-type pertinente ;
e) composition, par l'ordinateur de composition, d'une réponse composée à partir de ladite au moins une réponse type pertinente ;
f) optionnellement, validation et/ou modification de la réponse composée par un contrôleur ;
g) présentation de la réponse composée, optionnellement validée et/ou modifiée ;
la sélection à l'étape d) et la composition à l'étape e) étant déterminées conformément à des règles de composition d'un protocole de composition.

2. Procédé selon la revendication immédiatement précédente, dans lequel, à l'étape g), l'ordinateur de composition transmet la réponse composée, optionnellement validée et/ou modifiée, au téléphone mobile, et le téléphone mobile présente la réponse composée, optionnellement validée et/ou modifiée.

3. Procédé selon l'une quelconque des revendications précédentes, dans lequel le protocole de composition est commun à tous les patients d'un groupe de patients du professionnel des soins dentaires.

4. Procédé selon l'une quelconque des revendications précédentes, dans lequel, à l'étape c), la valeur de l'attribut dentaire qualifie ou quantifie la situation dentaire générale du patient cible et/ou une situation orthodontique.

5. Procédé selon la revendication immédiatement précédente, dans lequel, à l'étape c), la valeur de l'attribut dentaire qualifie ou quantifie un état d'un appareil orthodontique représenté sur l'image actualisée et/ou un état d'une dent du patient cible représentée sur l'image actualisée et/ou l'hygiène bucco-dentaire du patient.

6. Procédé selon l'une quelconque des revendications précédentes, dans lequel, à l'étape c), on effectue ladite analyse au moyen d'un algorithme d'intelligence artificielle.

7. Procédé selon l'une quelconque des revendications précédentes, dans lequel, avant l'étape e), le professionnel des soins dentaires sélectionne le protocole de composition dans une base de modèles de protocole et/ou crée le protocole de composition en sélectionnant un modèle de protocole dans une base de modèles de protocoles, puis en le modifiant.

8. Procédé selon l'une quelconque des revendications précédentes, dans lequel, avant l'étape e), le professionnel des soins dentaires configure le protocole de composition de manière à sélectionner une région de la bouche du patient pour que l'analyse à l'étape c) et/ou la sélection de la réponse-type pertinente à l'étape d) ne portent que sur ladite région.

9. Procédé selon l'une quelconque des revendications précédentes, dans lequel, à l'étape d), l'ordinateur de composition sélectionne au moins une réponse-type en fonction de données relatives au patient cible non déterminables par l'analyse de la situation dentaire de l'étape c).

10. Procédé selon la revendication immédiatement précédente, dans lequel, avant l'étape d), l'ordinateur évalue la réaction du patient cible suite à l'envoi d'une réponse composée antérieure générée lors d'une étape g) antérieure, puis, à l'étape d), sélectionne ladite au moins une réponse-type en fonction de l'évaluation de ladite réaction.

11. Procédé selon la revendication immédiatement précédente, dans lequel la réponse composée antérieure est une réponse générée lors d'un cycle d'étapes a) à g) antérieur, à partir d'une image actualisée antérieure, ou est une réponse générée lors d'une étape g) antérieure portant sur ladite image actualisée.

12. Procédé selon l'une quelconque des revendications précédentes, dans lequel au moins une règle de composition est configurée pour agir sur ladite composition en fonction de ladite au moins une réponse type pertinente sélectionnée à l'étape d), et/ou en fonction de données relatives au patient cible et/ou de données relatives au professionnel de soins dentaires.

13. Procédé selon la revendication immédiatement précédente, dans lequel la règle de composition est configurée pour agir sur ladite composition en fonction de données relatives au patient cible non déterminables par l'analyse de la situation dentaire de l'étape c).

14. Procédé selon l'une quelconque des revendications précédentes, dans lequel à l'étape f), le contrôleur est un professionnel des soins dentaires différent du professionnel des soins dentaires qui suit le patient cible, et dans lequel le contrôleur contrôle et/ou modifie des réponses composées pour des patients de plus de 5 professionnels des soins dentaires.

15. Procédé selon l'une quelconque des revendications précédentes, dans lequel l'ordinateur d'analyse et l'ordinateur de composition sont intégrés dans le téléphone mobile, ou dans lequel l'ordinateur d'analyse et/ou l'ordinateur de composition sont à distance du téléphone mobile.

16. Procédé selon l'une quelconque des revendications précédentes, dans lequel l'ordinateur de composition compose, suivant un protocole de composition identique ou différent de celui mis en oeuvre à l'étape d) et à l'étape e), un bilan médical en fonction de ladite valeur dudit au moins un attribut dentaire, et présente ledit bilan médical au professionnel de soins dentaires.
